(19) Europäisches Patentamt
European Patent Office
Office européen des brevets

(11) **EP 2 882 434 B1**

(12) **EUROPEAN PATENT SPECIFICATION**

(45) Date of publication and mention
of the grant of the patent:
**08.08.2018   Bulletin 2018/32**

(21) Application number: **13827217.4**

(22) Date of filing: **06.08.2013**

(51) Int Cl.:
*A61K 31/352* (2006.01)          *A61P 3/10* (2006.01)
*A61P 17/02* (2006.01)

(86) International application number:
**PCT/IB2013/056439**

(87) International publication number:
**WO 2014/024136 (13.02.2014 Gazette 2014/07)**

(54) **A METHOD OF MANAGING DIABETIC FOOT ULCERS, PRESSURE ULCERS, VENOUS LEG ULCERS AND ASSOCIATED COMPLICATION**

VERFAHREN FÜR DAS MANAGEMENT DIABETISCHER FUSSGESCHWÜRE, DRUCKGESCHWÜRE, VENÖSER BEINGESCHWÜRE UND ASSOZIIERTER KOMPLIKATIONEN

PROCÉDÉ DE GESTION D'ULCÈRES DIABÉTIQUES DU PIED, DE PLAIES DE PRESSION, D'ULCÈRES VEINEUX DE LA JAMBE ET DE COMPLICATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **07.08.2012   IN 2268MU2012**

(43) Date of publication of application:
**17.06.2015   Bulletin 2015/25**

(73) Proprietor: **Indus Biotech Private Limited
Pune 411 048, Maharashtra (IN)**

(72) Inventors:
• **BHASKARAN, Sunil
Maharashtra
Pune 411048 (IN)**

• **VISHWARAMAN, Mohan
Maharashtra
Pune 411048 (IN)**

(74) Representative: **Zacco Sweden AB
P.O. Box 5581
114 85 Stockholm (SE)**

(56) References cited:
| | |
|---|---|
| WO-A1-2006/030426 | WO-A1-2008/142702 |
| WO-A1-2012/014165 | WO-A2-2008/011173 |
| WO-A2-2009/020481 | US-A1- 2006 293 258 |
| US-A1- 2011 039 923 | US-A1- 2011 064 833 |

• **DATABASE WPI Derwent Publications Ltd., London, GB; AN 2012-A22565, XP003034219 WPAT & CN 102 274 514 A (TIANJIN MULTISTAR KONWA PHARMACEUTICAL SCIENCE AND TECHNOLOGY) 14 December 2011**

EP 2 882 434 B1

## Description

### TECHNICAL FIELD

The present disclosure is related to managing diabetic foot ulcers (DFUs,) pressure ulcers, venous leg ulcers and associated complications such as bacterial infection, gangrene, tissue necrosis, amputation, proximal limb loss, and septicaemia by administration of pharmaceutical composition comprising pentameric type A procyanidin, trimeric procyanidin and tetrameric procyanidin, optionally along with pharmaceutical excipient.

### BACKGROUND AND PRIOR ART

Catechins are polyphenolic plant metabolites which belong to the flavonoid family. Catechin and epicatechin are epimers, with (-)-epicatechin and (+)-catechin being the most common optical isomers found in nature. Procyanidins or condensed tannins are flavonoid oligomers whose building blocks are (+) -catechin and (-) - epicatechin. They are present abundantly in the plant kingdom in fruits, barks, leaves and seeds where they provide protection against light, oxidation and predators.

Procyanidins are found in many plants, mainly apples, pine bark, cinnamon bark, litchi pericarp, peanuts, grape seed, cocoa, grape skin, bilberry, cranberry, black currant, green tea and black tea. Based on the linkage between the successive monomeric units, procyanidins are classified as Types A, B or C polyphenols. Generally the linkage between successive monomeric units of procyanidins is between the 4th position of the 'upper' unit and the 8th position of the 'lower' unit, leading to a Type B procyanidin.

Alternatively, the linkage can occur between C4 of the 'upper' unit and C6 of the lower unit, leading to a Type C procyanidin. Type B and C polyphenols are abundantly seen in many botanical sources. When successive monomeric units are linked by an ether linkage between the C2 and C4 of the 'upper' unit and the oxygen at the C7 position and the C6/C8 positions (respectively) of the lower unit, a Type A procyanidin is formed.

Diabetes mellitus, or simply, Diabetes, is a group of diseases characterized by high blood glucose levels that result from defects in the body's ability to produce and/or use insulin. Diabetes is a disease associated with complications. One of the prominent complications associated with diabetes is diabetic foot ulceration which may lead to amputation and septicaemia. Journal of American Medical Association describes Diabetic Foot Ulcers (DFUs) as sores or wounds on the feet that occur in people with diabetes, a condition where blood sugar levels are abnormally high. Diabetic foot ulcers typically arise on a foot with poor nerve signalling and poor circulation, often caused by harmful pressure on parts of the foot.

People with diabetes have a 12-25% lifetime risk of developing a foot ulcer. The occurrence of chronic non-healing Diabetic Foot Ulcer (DFU) is around 15 % of all persons with Diabetes. Diabetic Foot Ulcer is a serious complication of Diabetes and precedes 84% of all diabetes related lower leg amputations. (S. Guo and L. A. DiPietro 2010). Foot ulcers cause substantial morbidity, impair quality of life, engender high treatment costs and are the most important risk factor for lower-extremity amputation. Unfortunately, treatment provided for foot ulcers is often inadequate; resulting in complications and unnecessarily extended healing times.

Diabetic neuropathy leads to decreased pain sensation in the late stages of Diabetes. Loss of pain and sensation occur in presence of sensory neuropathy and the patient continues to use the foot despite the presence of a foot ulcer. Peripheral vascular disease may lead to foot ischemia and can directly cause ischemic foot ulceration due to hyperglycaemia induced atherosclerosis. This further leads in narrowing and blockage of arteries that affects the RBC deformability and results in impaired blood and oxygen supply to the legs.

At this stage, increased blood glucose level, reduced oxygen and blood supply to the site of injury and increased infection impairs the normal wound healing stages by making the wound hypoxic. This hypoxia gets prolonged and elevates inflammatory phase resulting in increased formation of advanced glycation end products (AGEs) that impairs granulation tissue formation and epithelialisation.

Meanwhile, hyperglycemia induced deregulated cellular functions such as defects in leukocyte chemotaxis andphagocytosis impairs ability of host leukocytes to fight against bacterial infection. Bacterial infection tends to produce endotoxins which results in local edema and thrombosis which may further cause ischemic necrosis leading to formation of gangrenous tissue which may be either in the form of dry or wet gangrene. Wet gangrene is the most prevalent form as it occurs in the extremities due to arterial obstruction and severe bacterial infection of necrotic area.

A non-healing diabetic foot ulcer with highly elevated levels of bacteria due to necrosis can lead to Sepsis. Clinical practitioners often decide to do amputation of the diabetic foot ulcer area so as to avoid a fatal sepsis situation. However, the wound created by amputation may also result in a non-healing diabetic wound.

A pressure ulcer is an area of skin that breaks down when constant pressure is placed against the skin. Primary risk factor in the pathogenesis of the lesions is unrelieved pressure. Pressure ulcers develop when capillaries supplying the skin and subcutaneous tissues are compressed enough to impede perfusion, leading ultimately to tissue necrosis.

Commonly known as Bed sores, a pressure ulcer remains unhealed due to blood stasis in the affected area. Pressure ulcers are difficult to heal due to lack of blood supply and nutrition to the affected area. If not adequately treated, open pressure ulcers can become a source of pain, disability, infection and septicaemia.

Current treatments to treat DFUs, pressure ulcers and related complications are either systemic or topical applications. Systemic applications include use of antibiotics to treat the DFUs. As explained above, due to the intrinsic nature of DFU and pressure ulcer, drug circulation at the distal areas of the body is impaired and administration of antibiotics by oral or parenteral route may not be effective. So an antibiotic regimen may not be effective in the case of DFUs and pressure ulcer as the drug doesn't reach the affected areas resulting in necrosis and further complications. Similarly, topical applications of antibiotics are also not useful.

In topical applications, REGRANEX Gel 0.01% (becalpermin) cream has been used to treat DFUs. REGRANEX is a recombinant human platelet-derived growth factor manufactured by Healthpoint Biotherapeutics. Prescription information of REGRANEX product carries a warning stating that increased rate of mortality secondary to malignancy is observed in patients treated with 3 or more tubes of Regranex gel in a post marketing retrospective cohort study.

Advanced Tissue Sciences manufactured DERMAGRAFT, a skin substitute used to help in wound closure of DFUs. Plermin gel is a recombinant human platelet-derived growth factor recently launched by Dr. Reddy's Laboratories for the treatment of DFUs. These topical applications are unable to act as there is no systemic circulation providing oxygen and nutrients to DFU. Thus there is always a lack of oxygen and nutrients leading to the necrotic cell death causing septicaemia. Topical agents and dressings may reduce, but do not prevent, the inexorable progression of the injury to deeper layers. Regeneration cannot take place until equilibrium is reached; hence, healing is retarded.

Management options for pressure ulcers include topical applications, pressure reducing devices and use of antibiotics. Applications such as transparent films, hydrogels and hydrocolloid dressings act topically, but they are unable to provide oxygen and necessary nutrients to the wound. Pressure reducing devices are used to redistribute localised pressure but they are effective only as a preventive measure for pressure ulcers. They are not an effective solution for an already existing pressure ulcer. An antibiotic regimen is not effective in pressure ulcer because the drug doesn't reach the distal areas of wound further resulting in necrosis and further complications.

Compression therapy is the standard of care for venous ulcers. Methods include inelastic, elastic, and intermittent pneumatic compression. Contraindications to compression therapy include clinically significant arterial disease and uncompensated heart failure. Topical negative pressure, also called vacuum-assisted closure, has been shown to help reduce wound depth and volume compared with a hydrocolloid gel and gauze regimen for wounds of any etiology. The therapy generally has not been used in clinical practice because of the challenge in administering both topical negative pressure and a compression dressing on the affected leg.

A skin graft is a type of constitutive surgery that may be used to repair damage to the skin caused by gangrene. The surgery has some disadvantages such as textural and thickness differences, inability to place a supporting cartilage graft under the skin graft at the time of reconstitution, and additional donor site discomfort.

Other options include removal of dead tissue by surgery, use of antibiotics with limitations of surgical discomfort and limited oxygen and nutrition supply to the wound with continued spreading of infection. Hyperbaric oxygen therapy is another option to overcome the influence of tissue hypoxia but the treatment has limitation of availability and it requires mechanism oriented translational research to evaluate the risk: benefit ratio of systemic oxygen therapy in treatment.

Bhaskaran et al. (US 2011/0039923 A1) discloses a composition comprising pentameric procyanidin flavonoid of concentration ranging from about 55% w/w to about 99% w/w, trimers and tetramers each at a concentration ranging from about 0.5% w/w to about 35% w/w. This document also discloses a process for preparation of the said composition. Further, this document teaches use of the said composition for treatment and management of HIV infection, AIDS and Influenza virus infection. However, this document does not suggest or teach the use of the said composition in healing diabetic foot ulcers through oral administration of composition, to prevent bacterial infection leading to gangrene in diabetic foot ulcers and reduce the chances of amputation and limb loss.

Bhaskaran et al. (WO2012/014165 A1) discloses a method of managing broncho-constrictive condition, said method comprising act of administering a composition comprising pentameric type A procyanidin ranging from about 55 % w/w to about 99% w/w, trimeric procyanidin and tetrameric procyanidin are each at concentration ranging from about 0.5 % w/w to about 35 % w/w; optionally along with one or more pharmaceutical excipients. It also discloses that broncho-constrictive condition is selected from group comprising allergic rhinitis, asthma and chronic obstructive pulmonary disease or any combinations thereof. However, this document does not suggest or teach the use of the said composition in healing in diabetic foot ulcers.

## STATEMENT OF THE DISCLOSURE

Accordingly, the present disclosure relates to a method of managing diabetic foot ulcers (DFUs), pressure ulcers, venous leg ulcers and associated complication, said method comprising act of administering composition comprising pentameric type A procyanidin, trimeric procyanidin and tetrameric procyanidin, optionally along with pharmaceutically acceptable excipient, to subject in need thereof.

## BRIEF DESCRIPTION OF THE ACCOMPANYING FIGURES

In order that the disclosure may be readily understood and put into practical effect, reference will now be made to exemplary embodiments as illustrated with reference to the accompanying figures. The figures together with a detailed description below, are incorporated in and form part of the specification, and serve to further illustrate the embodiments and explain various principles and advantages, in accordance with the present disclosure where:

**Figure 1** presents a diabetic foot ulcer which resulted in gangrene toe and amputation of two fingers.
**Figure 2** presents efficacy of the instant composition in managing Diabetic foot ulcer on the basis of photographic evidence taken during interval of the base line.
**Figure 3** presents efficacy of the instant composition in managing Diabetic foot ulcer on the basis of photographic evidence taken during the interval of about 2 months.
**Figure 4** presents efficacy of the instant composition in managing Diabetic foot ulcer on the basis of photographic evidence taken during the interval of about 3 months.
**Figure 5** presents efficacy of the instant composition in managing Diabetic foot ulcer on the basis of photographic evidence taken during the interval of about 7 months.
**Figure 6** presents efficacy of the instant composition in managing Diabetic foot ulcer on the basis of photographic evidence taken during the interval of about 11 months.
**Figure 7** presents a graph of % wound closure versus time on Day 13 after wound creation.
**Figure 8** presents a graph of linear regression analysis for (a) normal group (b) DW control (c) instant composition group of 10 mg/kg (d) instant composition group of 30 mg/kg and (e) instant composition group of 100 mg/kg.

## DETAILED DESCRIPTION OF THE DISCLOSURE

The present disclosure relates to a method of managing diabetic foot ulcers (DFUs), pressure ulcers, venous leg ulcers and associated complication; said method comprising act of administering composition comprising pentameric type A procyanidin, trimeric procyanidin and tetrameric procyanidin, optionally along with pharmaceutically acceptable excipient, to subject in need thereof.

In an embodiment of the present disclosure, the pentameric type A procyanidin is at concentration ranging from about 55 % w/w to about 99% w/w, the trimeric procyanidin and the tetrameric procyanidin are each at concentration ranging from about 0.5 % w/w to about 35 % w/w; and the pharmaceutically acceptable excipient is at concentration ranging from about 0.5% to about 99.9%.

In another embodiment of the present disclosure, the pentameric type A procyanidin is at concentration ranging from about 80 % w/w to about 90% w/w, the trimeric procyanidin and the tetrameric procyanidin are each at concentration ranging from about 0.5 % w/w to about 20 % w/w.

In yet another embodiment of the present disclosure, the associated complication is selected from group comprising bacterial infection, gangrene, tissue necrosis, amputation, proximal limb loss and septicemia or any combinations thereof.

In still another embodiment of the present disclosure the pharmaceutically acceptable excipient is selected from group comprising gum, granulating agent, binder, lubricant, disintegrating agent, sweetening agent, additive, solvent, glidant, anti-adherent, anti-static agent, anti-oxidant, surfactant, viscosity enhancer, plant cellulosic material, coloring agent, flavoring agent, coating agent, plasticizer, preservative, suspending agent, emulsifying agent and spheronization agent or any combinations thereof.

In still another embodiment of the present disclosure, the composition is formulated into dosage forms selected from group comprising solid oral formulation, liquid oral formulation, parenteral formulation, phytoceutical, nutraceutical and medicinal food or any combination thereof.

In still another embodiment of the present disclosure, the solid oral formulation is selected from group comprising tablet, capsule, troche, lozenge, dispersible powder, dispersible granule or any combinations thereof.

In still another embodiment of the present disclosure, the liquid oral formulation is selected from group comprising aqueous or oily suspension, emulsion, drops, emulsion in hard or soft gel capsule, syrup, elixir or any combinations thereof.

In still another embodiment of the present disclosure, the parenteral formulation is selected from group comprising intravenous injection, intramuscular injection, intramuscular depot, subcutaneous injection, percutaneous injection or any combinations thereof.

In still another embodiment of the present disclosure, the composition is administered at daily dose ranging from about 1 mg/kg to about 100 mg/kg, preferably ranging from about 10mg/kg to about 25mg/kg of body weight of said subject.

In still another embodiment of the present disclosure, the subject is a mammal, including human beings.

In another embodiment of present disclosure, administration of an oral pharmaceutical composition comprising pentameric type A procyanidin, trimeric procyanidin and tetrameric procyanidin, optionally along with pharmaceutically acceptable excipient(s) is carried out.

In another embodiment of present disclosure, other oral formulations like tablets, liquid orals, and powders or granules are also prepared by using other excipients selected from list comprising excipients.

In still another embodiment of the present disclosure, the instant composition refers to pentameric type A procyanidin at concentration ranging from about 55 % w/w to about 99% w/w, the trimeric procyanidin and the tetrameric procyanidin are each at concentration ranging from about 0.5 % w/w to about 35 % w/w; and the pharmaceutically acceptable excipient is at concentration ranging from about 0.5 % to about 99.9%.

In an embodiment of the present disclosure, the term "managing" or "management" includes preventing, treating and healing of a disease condition or disorder or ill effects or side effects. The term also encompasses maintenance of the optimum state and prevention of the further progress in the disease condition or disorder or ill effects or side effects.

In another embodiment of the present disclosure, the instant composition is formulated into a suitable dosage formulation for managing diabetic foot ulcers (DFUs), pressure ulcers, venous leg ulcers and associated complication, using a daily dose ranging from about 1 mg/kg to about 100 mg/kg of body weight of subject, preferably ranging from about 10 mg/kg to about 25 mg/kg of body weight of subject.

In an embodiment of the present disclosure, the dose of the instant composition for subjects is calculated according to the USFDA guidelines for the industry.

In an embodiment of the present disclosure, animal studies are carried out with doses of the instant composition at 10 mg/kg, 30 mg/kg, and 100 mg/kg twice daily.

The instant disclosure is further elaborated with the help of following examples. However, these examples should not be construed to limit the scope of the present disclosure.

## EXAMPLES

### Example 1: Formulation of instant composition

The instant composition comprising type A pentameric procyanidin flavonoid of concentration ranging from about 55 % w/w to about 99% w/w, trimers and tetramers of pro cyanidin flavonoid each at concentration ranging from about 0.5 % w/w to about 35 % w/w is formulated into capsules by blending with about 2% w/w of micro crystalline cellulose, about 0.5% w/w of crospovidone and about 0.2% w/w of magnesium stearate. This mixture is filled in capsules.

Similar formulation of the instant composition is prepared by addition of appropriate excipient(s) selected from list comprising: granulating agent, binding agent, lubricating agent, disintegrating agent, sweetening agent, glidant, anti-adherent, anti-static agent, surfactant, anti-oxidant, gum, coating agent, coloring agent, flavouring agent, coating agent, plasticizer, preservative, suspending agent, emulsifying agent, plant cellulosic material and spheronization agent or any combination thereof.

In an embodiment of the instant disclosure, Saccharides and their derivatives, Cellulose or Stearic acid are used as pharmaceutically acceptable excipient.

The type of formulation is selected from group comprising solid oral formulation, liquid oral formulation, parenteral formulation, phytoceutical, nutraceutical and medicinal foods or any combinations thereof.

In an embodiment of the present disclosure, the solid oral formulation is selected from group comprising tablet, capsule, troche, lozenge, dispersible powder, dispersible granule or any combinations thereof.

In an embodiment of the present disclosure, the liquid oral formulation is selected from group comprising aqueous or oily suspension, emulsion, drop, emulsion in hard or soft gel capsule, syrup, elixir or any combinations thereof.

In an embodiment of the present disclosure, the parenteral formulation is selected from group comprising intravenous injection, intramuscular injection, intramuscular depot, subcutaneous injection, percutaneous injection or any combinations thereof.

In an embodiment of present disclosure, a person skilled in the art would be able to achieve similar efficacy of the present composition by using other formulations as listed above. Depending on the route of administration, different excipients/carriers are used.

Those skilled in art will know to choose a suitable formulation of the instant composition for managing diabetic foot ulcers (DFUs), pressure ulcers, venous leg ulcers and associated complication, using a daily dose range from about 1 mg/kg to about 100 mg/kg of body weight of subject or preferably, a daily dose range from about 10 mg/kg to about 25 mg/kg of body weight of subject.

**Example 2: Evaluation of Diabetic foot ulcer healing activity of instant composition in Streptozotocin (STZ) induced diabetic rats with foot ulcers**

Diabetes is induced in Sprague-Dawley rats (age about 6-7 weeks, weight - about 180-200 g) using the standard procedure of an intraperitoneal (i.p) injection of Streptozotocin (STZ) (about 50 mg/kg, i.p). After about 48 hours, the rats with glucose level greater than 300 mg/dl (indicative of diabetes) are selected for study.

After about 14 days of STZ administration, a wound is created and the day is defined as day 0. The wound is created on right hind-paw (foot) of each rat. Each rat is anesthetized with intraperitoneal injection of about 80 mg/kg ketamine. A rectangular pattern is marked on the dorsal surface of the right hind paw (foot) using a flexible transparent plastic template, and then a layer of skin in full thickness with standard area of about 2 mm $\times$ 5 mm is removed.

The rats are either administered with vehicle - Distilled water, about 10 mg /kg body weight (diabetic wound, DW control) or instant composition (about 10 mg/kg, about 30 mg/kg or about 100 mg/kg, oral) for about 13 days after induction of diabetic wound. Separate group of rats without diabetes induction is also maintained with wound creation (defined as normal wound control).

The following parameters are measured on various days after wound creation:

- body weight (g),
- serum glucose level (mg/dl), and
- wound area (mm$^2$).

On 13$^{th}$ day of treatment, histological examination is carried out on wound tissue and sciatic nerve of few rats after they are sacrificed.

On day 0, 3, 6, 9, and 13, the wounds are digitally photographed using a Nikon S9100. The area of the wound (WA) is calculated by image analysis software (ImageJ, NIH, USA). The percent (%) wound closure is calculated by following formula:

$$\% \text{ Wound closure} = -100 \times [(WA2) - (WA1)/(WA1)]$$

Where,

WA1 = Area of wound on day 0 (maximum wound size); and
WA2 = Area of wound on day 3, 6, 9 or 13.

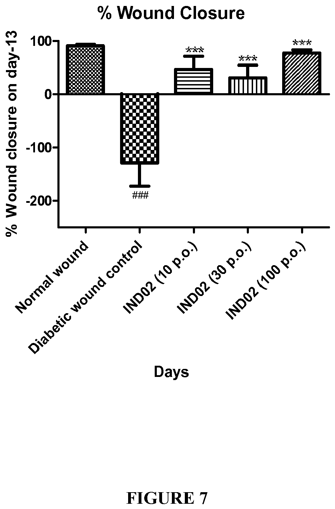

The graph of % wound closure versus time in days from wound creation is plotted in software (Graphpad Prism v 5) and linear regression analysis is performed [Figure 7]. The value of X at Y=50% is taken as CT50 (half-closure time, time taken to close the wound by 50%).

**Results:**

**Table 1: Effect of treatment of instant composition on % wound closure in diabetic wound**

| Days after wound creation | % Wound closure (mm$^2$) - Mean $\pm$ SEM | | | | |
|---|---|---|---|---|---|
| | Normal wound | DW control | DW+ Instant composition (10mg/kg, orally) | DW+ Instant composition (30mg/kg, orally) | DW+ Instant composition (100mg/kg, orally) |
| 3 | 0.00 $\pm$ 0.00 | 0.00 $\pm$ 0.00 | 0.00 $\pm$ 0.00 | 0.00 $\pm$ 0.00 | 0.00 $\pm$ 0.00 |
| 6 | -9.52 $\pm$ 14.89 | -7.12 $\pm$ 15.06 | 30.26 $\pm$ 6.67 | 40.42 $\pm$ 6.39 | 15.40 $\pm$ 7.45 |
| 9 | 69.78 $\pm$ 8.68 | -85.36 $\pm$ 41.84$^{\#\#\#}$ | 46.38 $\pm$ 14.39*** | 40.94 $\pm$ 11.24*** | 44.68 $\pm$ 11.62*** |

(continued)

| Days after wound creation | % Wound closure (mm$^2$) - Mean $\pm$ SEM | | | | |
|---|---|---|---|---|---|
| | Normal wound | DW control | DW+ Instant composition (10mg/kg, orally) | DW+ Instant composition (30mg/kg, orally) | DW+ Instant composition (100mg/kg, orally) |
| 13 | 91.08 $\pm$ 2.99 | -129.01 $\pm$ 44.27$^{\#\#\#}$ | 47.49 $\pm$ 24.49*** | 31.34 $\pm$ 25.53*** | 77.47 $\pm$ 5.98*** |
| n = 4 to 6, wherein n = the number of rats in each group. DW - Diabetic wound control. | | | | | |

The treatment using instant composition is administered twice a day for about 13 days starting from wound creation in diabetic rats. Data is analyzed by two way ANOVA followed by Bonferroni's test.

$*P < 0.05$, $**P < 0.01$, $***P < 0.001$ as compared to DW control group and $^{\#}P < 0.05$, $^{\#\#}P < 0.01$, $^{\#\#\#}P < 0.001$ as compared to normal group.

- Positive (+ve) sign indicates reduced wound size (closure of wound),
- Negative (-ve) sign indicates increased size of wound.

**Conclusion:** Wound closure is negative for diabetic wound control which indicates aggravation of wound. Wound closure is positive for Normal and instant composition treated group, which indicates healing of wound. Normal wound closure is about 91.08% in about 13 days. Diabetic wound untreated (control group) shows aggravation by increase in wound size to about 129%.

Diabetic wounds in instant composition treated group show % wound closure by about 47%, about 31% and about 77% at about 10 mg/kg, about 30 mg/kg and about 100 mg/kg, oral, twice daily respectively in the same period. The instant composition treatment shows significant closure of diabetic wound (wound healing) after treatment for about 9 days and onwards.

From this experiment it is important to note that the natural mechanism of healing of a normal wound is demonstrated in the control group. But the natural process of healing is impaired or abrogated in Diabetic wound untreated (control group), and the diabetic wound aggravates substantially. But in the instant composition treated groups, the aggravation of the diabetic wound is not seen and the healing process has started. The instant composition groups show very good healing process compared to the control group of Diabetic wound.

**Table 2: Effect of treatment by instant composition on slope of regression line and CT50 (half-closure time)**

| Parameter | Normal wound | DW control | Instant composition (10 mg/kg, orally) | Instant composition (30 mg/kg, orally) | Instant composition (100 mg/kg, orally) |
|---|---|---|---|---|---|
| **Slope** | 5.94 $\pm$ 1.84 | -8.43 $\pm$ 1.84 | 4.12 $\pm$ 0.58 | 3.45 $\pm$ 0.93 | 5.09 $\pm$ 0.81 |
| **CT50 (days)** | 8.40 | -5.92 | 12.13 | 14.48 | 9.82 |
| n = 4 to 6, wherein n = the number of rats in each group. DW - Diabetic wound control. | | | | | |

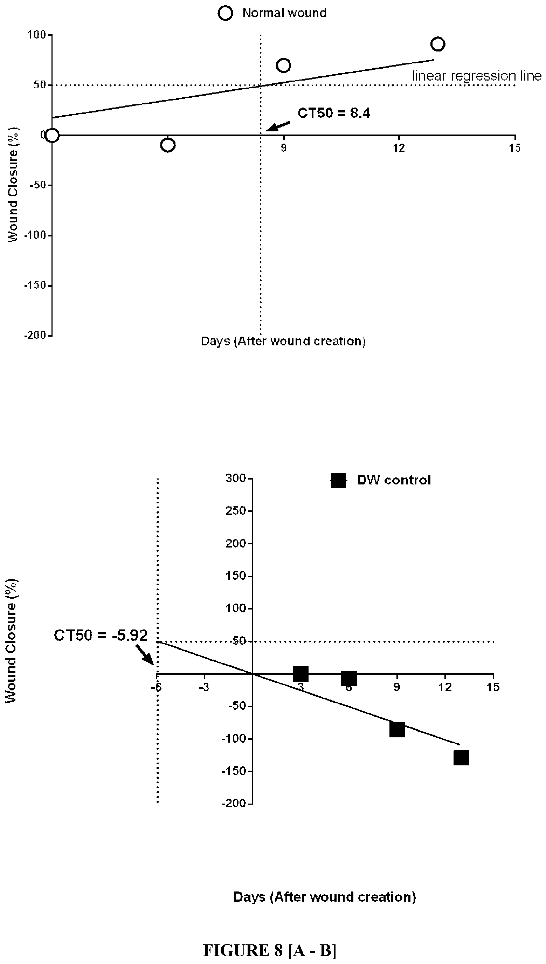

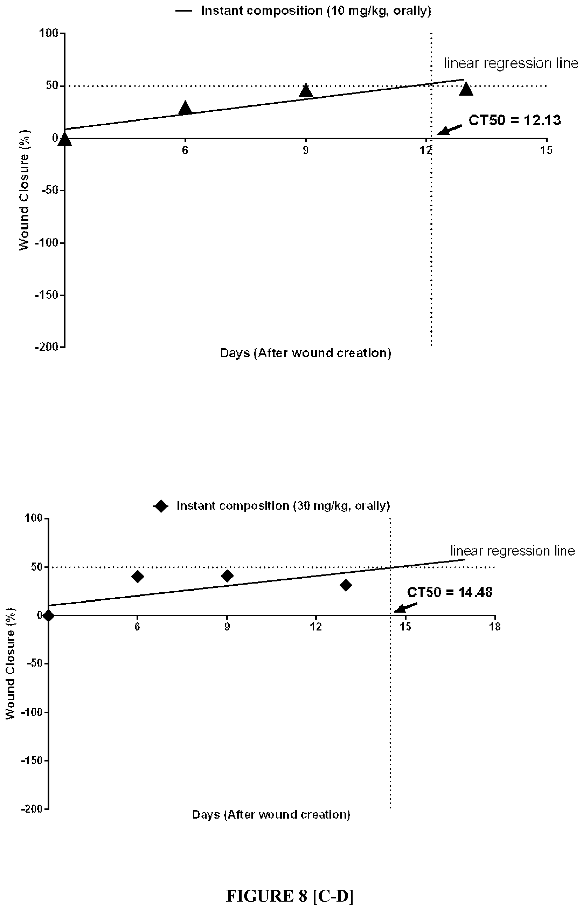

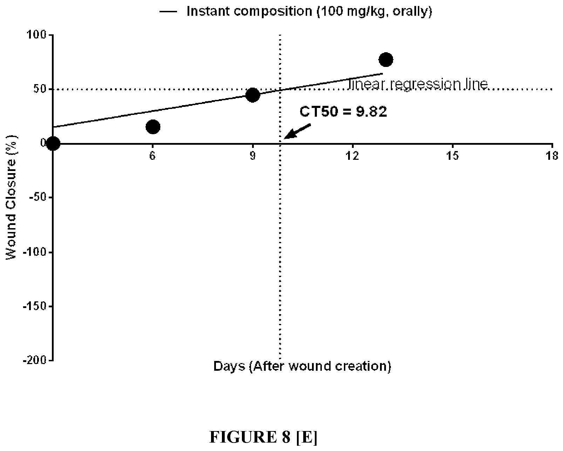

The graph of % wound closure versus time in days is plotted in software (Graphpad Prism v 5) and linear regression analysis is performed. The value of X at Y=50% is taken as CT50 (half-closure time, Time taken to close the wound by 50%). Positive (+ve) sign of slope and CT50 indicate closure of wound, Negative (-ve) sign of slope and CT50 indicate no closure and hence aggravation of wound. The graph is presented in figure 8.

**Conclusion:** The positive slope of regression line indicates rate of wound closure (healing). Slope of normal group (figure 8a) is positive (indicating closure of wound) whereas slope of DW control (figure 8b) group is negative (indicating no healing, aggravation of wound). The instant composition treatment shows positive slope of line which indicates wound healing.

CT50 (time required to close the wound by 50%) of about 8.4 days shows wound healing in normal group. However CT50 of about 5.92 days shows no closure and aggravation of wound in DW group. This provides us a conclusion that there is need of specific treatment targeting wounds in diabetic patients. The instant composition group of about 10 mg/kg (figure 8c), about 30 mg/kg (figure 8d) and about 100 mg/kg (figure 8e) shows CT 50 of about 12.13 days, about 14.48 days and about 9.82 days respectively. This demonstrates the efficacy of instant composition in healing diabetic wounds (Diabetic foot ulcer).

## Example 3: Effect of instant composition in a subject suffering from Diabetic Foot Ulcer

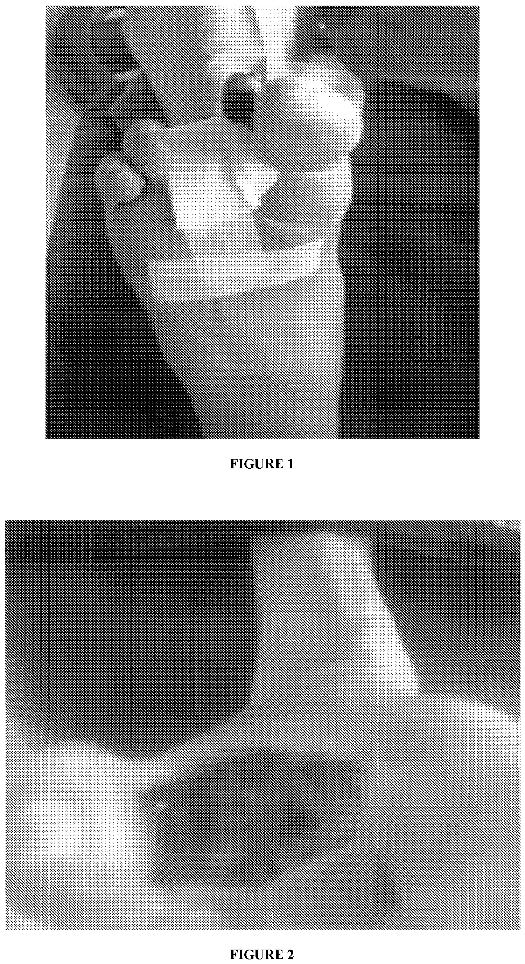

A study is conducted to assess the efficacy of the instant composition in a human subject suffering from Diabetic Foot ulcer. The selected subject (Male, age 85 years) is a chronic diabetic patient diagnosed with Type 2 Diabetes Mellitus for more than 25 years. The subject is on insulin along with multiple oral anti-diabetic drugs. The subject developed a diabetic foot ulcer which resulted in gangrene toe and amputation of two fingers (Figure 1).

The amputation wound further becomes a non-healing ulcer with necrotic environment in the wound area. At the start of the study, the subject is at a greater risk of further amputation so as to reduce progress of septic condition.

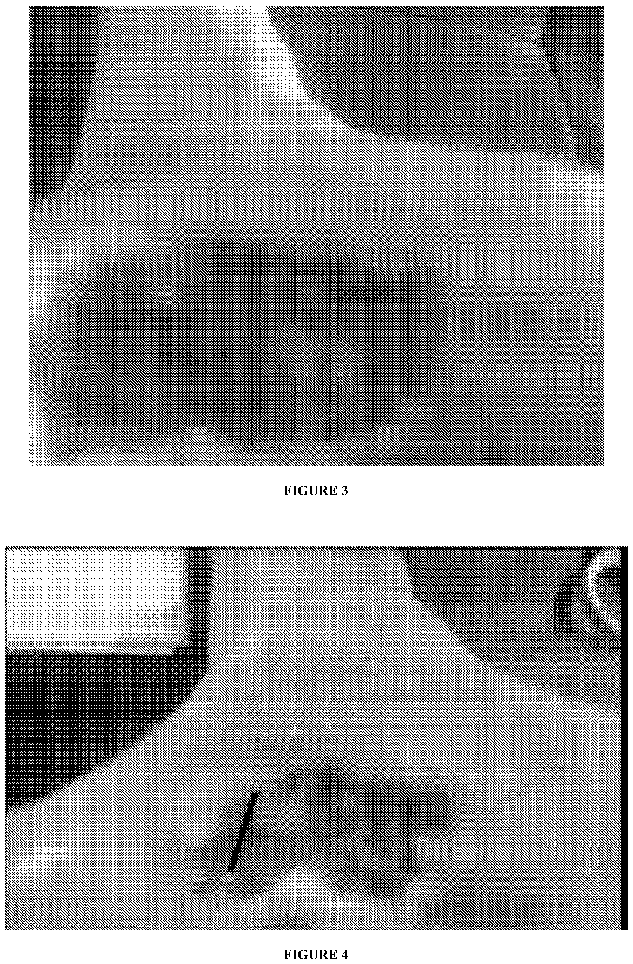

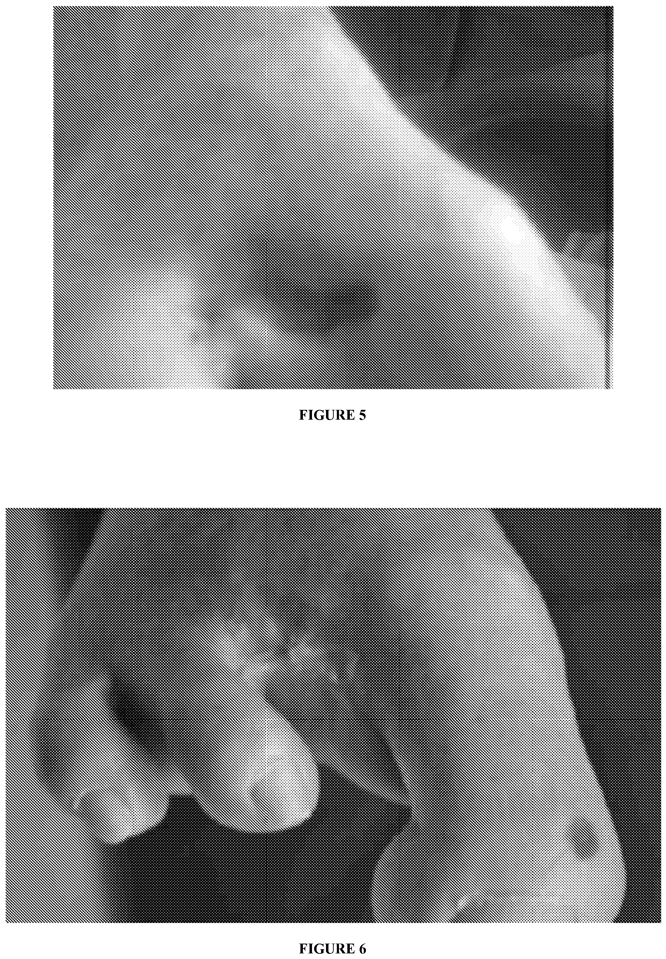

The dosage is calculated according to the USFDA guidelines for the industry as per the subject's weight and the subject is given capsules of the instant composition at dose of about 300 mg twice daily for a period of about 11 months. The efficacy of the instant composition is analysed on the basis of photographic evidence taken during intervals, such as base line (Figure 2), about 2 months (Figure 3), about 3 months (Figure 4), about 7 months (Figure 5) and about 11 months (Figure 6).

Following the initiation of administration of the instant composition; the subject reports keratinisation of the diabetic foot ulcer and gradual reduction of necrosis. Also, this treatment avoids any additional need for amputation and any complications leading to septicaemia. Photographic evidence [in figures 1 to 6] shows that the Diabetic Foot Ulcer has started the process of healing and in about 11 months time the complete bridging of the ulcer happens. It is observed that the ulcer has healed completely and after completion of treatment no further amputation is required, thus the treatment has succeeded to save further proximal limb loss.

## Example 4: Effect of instant composition in a subject suffering from pressure ulcer

A study is conducted to assess the efficacy of the instant composition in a human subject suffering from pressure ulcer. The selected subject (Female, age 80) had undergone a hip replacement surgery and is hospitalised. The subject developed bed sore on lower back area of the body and the size of the ulcer is about 2 inches in diameter and about 1.5 inches in depth. At the start of the study, pressure ulcer has almost reached the backbone of the subject.

The dosage for the subject is calculated according to the USFDA guidelines for the industry, as per the body weight of the subject and the subject is given capsules of the instant composition at a dose of about 300 mg twice daily for a period of about 9 months. The efficacy of the instant composition is analysed on the basis of measurement of the pressure ulcer during intervals such as baseline, about 2 months, about 4 months, about 6 months and about 8 months.

Following the initiation of administration of the instant composition, the subject reported keratinisation of the ulcer and gradual reduction of necrosis. The table below shows that the pressure ulcer has started the process of healing and in about 6 months time the complete bridging of the ulcer has happened. After about 8 months, the subject reported complete closure of the pressure ulcer.

**Table 3: Effect of treatment by instant composition on wound closure of subject with pressure ulcer**

| Serial | Base line | 2 Months | 4 months | 6 months | 8 months |
|---|---|---|---|---|---|
| Diameter of the Pressure ulcer | 2 inch | 1.9 inch | 1.2 inch | 0.6 inch | Complete closure of the ulcer |
| Depth of the pressure ulcer | 1.5 inch | 1.1 inch | 0.7 inch | 0.3 inch | Complete closure of the ulcer |

Thus, it can be derived from the results of the above table that administration of the instant composition to the subject has avoided any complications leading to septicaemia.

## Claims

1. A composition comprising pentameric type A procyanidin, trimeric procyanidin and tetrameric procyanidin, optionally along with pharmaceutically acceptable excipient for use in managing diabetic foot ulcers (DFUs), pressure ulcers, venous leg ulcers and associated complication.

2. The composition for use as claimed in claim 1, wherein the pentameric type A procyanidin is at concentration ranging from about 55 % w/w to about 99% w/w, the trimeric procyanidin and the tetrameric procyanidin are each at concentration ranging from about 0.5 % w/w to about 35 % w/w; and the pharmaceutically acceptable excipient is at concentration ranging from about 0.5% to about 99.9 %.

3. The composition for use as claimed in claim 2, wherein the pentameric type A procyanidin is at concentration ranging from about 80 % w/w to about 90% w/w, the trimeric procyanidin and the tetrameric procyanidin are each at concentration ranging from about 0.5 % w/w to about 20 % w/w.

4. The composition for use as claimed in Claim 1, wherein the associated complication is selected from group comprising infection, gangrene, tissue necrosis, amputation and septicemia or any combinations thereof.

5. The composition for use as claimed in Claim 1, wherein the pharmaceutically acceptable excipient is selected from group comprising gum, granulating agent, binder, lubricant, disintegrating agent, sweetening agent, additive, solvent, glidant, anti-adherent, anti-static agent, anti-oxidant, surfactant, viscosity enhancer, plant cellulosic material, coloring agent, flavoring agent, coating agent, plasticizer, preservative, suspending agent, emulsifying agent and spheronization agent or any combinations thereof.

6. The composition for use as claimed in claim 1, wherein the composition is formulated into dosage forms selected from group comprising, solid oral formulation, liquid oral formulation, parenteral formulation, phytoceutical, nutraceutical and food stuff or any combinations thereof.

7. The composition for use as claimed in claim 6, wherein the solid oral formulation is selected from group comprising tablet, capsule, troche, lozenge, dispersible powder, dispersible granule or any combinations thereof.

8. The composition for use as claimed in claim 6, wherein the liquid oral formulation is selected from group comprising aqueous or oily suspension, emulsion, drop, emulsion in hard or soft gel capsule, syrup, elixir or any combinations thereof.

9. The composition for use as claimed in claim 6, wherein the parenteral formulation is selected from group comprising intravenous injection, intramuscular injection, intramuscular depot, subcutaneous injection, percutaneous injection or any combinations thereof.

10. The composition for use as claimed in claim 1, wherein the composition is administered to a subject in need thereof, at daily dose ranging from about 1 mg/kg to about 100 mg/kg of body weight of said subject, preferably ranging from about 10mg/kg to about 25mg/kg of body weight of said subject.

11. The composition for use as claimed in claim 10, wherein the subject is a mammal, including human beings.

**Patentansprüche**

1. Zusammensetzung umfassend pentamerisches Typ A Prozyanidin, trimerisches Prozyanidin und tetramerisches Prozyanidin, eventuell zusammen mit einem pharmazeutisch verträglichen Hilfsstoff zur Verwendung beim Handhaben von diabetischen Fußgeschwüren (DFUs), Druckgeschwüren, venösen Beingeschwüren und einer damit verbundenen Komplikation.

2. Zusammensetzung zur Verwendung nach Anspruch 1, wobei das pentamerische Typ A Prozyanidin in einer Konzentration im Bereich von ca. 55 % Gew./Gew. bis ca. 99 % Gew./Gew. ist, das trimerische Prozyanidin und das tetramerische Prozyanidin jeweils in einer Konzentration im Bereich von ca. 0,5 % Gew./Gew. bis ca. 35 % Gew./Gew. sind; und der pharmazeutisch verträgliche Hilfsstoff in einer Konzentration im Bereich von ca. 0,5 % bis ca. 99,9 % ist.

3. Zusammensetzung zur Verwendung nach Anspruch 2, wobei das pentamerische Typ A Prozyanidin in einer Konzentration im Bereich von ca. 80 % Gew./Gew. bis ca. 90 % Gew./Gew. ist, das trimerische Prozyanidin und das tetramerische Prozyanidin jeweils in einer Konzentration im Bereich von ca. 0,5 % Gew./Gew. bis ca. 20 % Gew./Gew. sind.

**4.** Zusammensetzung zur Verwendung nach Anspruch 1, wobei die damit verbundene Komplikation aus der Gruppe bestehend aus Infektion, Wundbrand, Gewebenekrose, Amputation und Blutvergiftung oder jeglicher Kombination davon ausgewählt ist.

**5.** Zusammensetzung zur Verwendung nach Anspruch 1, wobei der pharmazeutisch verträgliche Hilfsstoff aus der Gruppe bestehend aus Gummi, Granuliermittel, Bindemittel, Schmiermittel, zersetzendem Mittel, Süßstoff, Zusatzstoff, Lösungsmittel, Gleitmittel, Antihaftmittel, Antistatikum, Antioxidans, Tensid, Viskositätsverbesserer, Pflanzenzellulosematerial, Farbstoff, Aromastoff, Beschichtungsmittel, Weichmacher, Konservierungsmittel, Suspensionsmittel, Emulgator und Sphäronisationsmittel oder jeglicher Kombination davon ausgewählt ist.

**6.** Zusammensetzung zur Verwendung nach Anspruch 1, wobei die Zusammensetzung in Darreichungsformen formuliert ist, ausgewählt aus der Gruppe bestehend aus fester oraler Formulierung, flüssiger oraler Formulierung, parenteraler Formulierung, phytozeutischen Mitteln, nutrazeutischen Mitteln und Nahrungsmitteln oder jeglicher Kombination davon.

**7.** Zusammensetzung zur Verwendung nach Anspruch 6, wobei die feste orale Formulierung aus der Gruppe bestehend aus Tablette, Kapsel, Pastille, Lutschtablette, dispergierbarem Pulver, dispergierbaren Körnchen oder jeglicher Kombination davon ausgewählt ist.

**8.** Zusammensetzung zur Verwendung nach Anspruch 6, wobei die flüssige orale Formulierung aus der Gruppe bestehend aus Wasser- oder Ölsuspension, Emulsion, Bonbon, Emulsion in einer harten oder weichen Gelkapsel, Sirup, Heiltrank oder jeglicher Kombination davon ausgewählt ist.

**9.** Zusammensetzung zur Verwendung nach Anspruch 6, wobei die parenterale Formulierung aus der Gruppe bestehend aus intravenöser Injektion, intramuskulärer Injektion, intramuskulärem Depot, subkutaner Injektion, perkutaner Injektion oder jeglicher Kombination davon ausgewählt ist.

**10.** Zusammensetzung zur Verwendung nach Anspruch 1, wobei die Zusammensetzung an ein Individuum mit einem Bedarf dafür in einer Tagesdosis im Bereich von ca. 1 mg/kg bis ca. 100 mg/kg des Körpergewichts des Individuums, vorzugsweise im Bereich von ca. 10mg/kg bis ca. 25mg/kg des Körpergewichts des Individuums, verabreicht wird.

**11.** Zusammensetzung zur Verwendung nach Anspruch 10, wobei das Individuum ein Säugetier, einschließlich Menschen, ist.

**Revendications**

**1.** Composition comprenant de la procyanidine de type A pentamérique, de la procyanidine trimérique et de la procyanidine tétramère, facultativement avec un excipient pharmaceutiquement acceptable à utiliser dans la gestion des ulcères du pied diabétique (UPD), des ulcères de pression, des ulcères de jambe veineux et des complications associées.

**2.** Composition pour l'utilisation selon la revendication 1, dans laquelle la procyanidine de type A pentamérique est à une concentration allant d'environ 55% en poids à environ 99% en poids, la procyanidine trimérique et la procyanidine tétramère sont chacune à une concentration allant d'environ 0,5% en poids à environ 35% en poids ; et l'excipient pharmaceutiquement acceptable est à une concentration allant d'environ 0,5% à environ 99,9%.

**3.** Composition pour l'utilisation selon la revendication 2, dans laquelle la procyanidine de type A pentamérique est à une concentration allant d'environ 80% en poids à environ 90% en poids, la procyanidine trimérique et la procyanidine tétramère sont chacune à une concentration allant d'environ 0,5% en poids à environ 20% en poids.

**4.** Composition pour l'utilisation selon la revendication 1, dans laquelle la complication associée est choisie dans le groupe comprenant l'infection, la gangrène, la nécrose tissulaire, l'amputation et la septicémie ou toute combinaison de celles-ci.

**5.** Composition pour l'utilisation selon la revendication 1, dans laquelle l'excipient pharmaceutiquement acceptable est choisi dans le groupe comprenant gomme, agent de granulation, liant, lubrifiant, agent désintégrant, édulcorant, additif, solvant, glissant, anti-adhérent, agent anti-statique, anti-oxydant, agent tensioactif, activateur de viscosité, matière cellulosique végétale, agent colorant, agent aromatisant, agent de revêtement, plastifiant, conservateur, agent de suspension, agent émulsifiant et agent de sphéronisation ou toute combinaison de ceux-ci.

6. Composition pour l'utilisation selon la revendication 1, dans laquelle la composition est formulée en des formes posologiques choisies parmi un groupe comprenant une formulation orale solide, une formulation orale liquide, une formulation parentérale, une substance phytoceutique, nutraceutique et alimentaire ou toute combinaison de celles-ci.

7. Composition pour l'utilisation selon la revendication 6, dans laquelle la formulation orale solide est choisie parmi un groupe comprenant un comprimé, une capsule, une troche, une pastille, une poudre dispersible, un granule dispersible ou toute combinaison de ceux-ci.

8. Composition pour l'utilisation selon la revendication 6, dans laquelle la formulation orale liquide est choisie parmi un groupe comprenant une suspension aqueuse ou huileuse, une émulsion, une goutte, une émulsion dans une gélule dure ou molle, un sirop, un élixir ou toute combinaison de celles-ci.

9. Composition pour l'utilisation selon la revendication 6, dans laquelle la formulation parentérale est choisie dans le groupe comprenant une injection intraveineuse, une injection intramusculaire, un dépôt intramusculaire, une injection sous-cutanée, une injection percutanée ou toute combinaison de celles-ci.

10. Composition pour l'utilisation selon la revendication 1, dans laquelle la composition est administrée à un sujet en ayant besoin, à une dose journalière allant d'environ 1mg/kg à environ 100 mg/kg de poids corporel dudit sujet, de préférence allant d'environ 10mg/kg à environ 25mg/kg de poids corporel dudit sujet.

11. Composition pour l'utilisation selon la revendication 10, dans laquelle le sujet est un mammifère, y compris des êtres humains.

**FIGURE 1**

**FIGURE 2**

FIGURE 3

FIGURE 4

FIGURE 5

FIGURE 6

**FIGURE 7**

FIGURE 8 [A - B]

EP 2 882 434 B1

FIGURE 8 [C-D]

17

FIGURE 8 [E]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110039923 A1, Bhaskaran
- WO 2012014165 A1, Bhaskaran